(19) Europäisches Patentamt
European Patent Office
Office européen des brevets

(11) Publication number : 0 449 453 B1

(12) # EUROPEAN PATENT SPECIFICATION

(45) Date of publication of patent specification : **03.11.93 Bulletin 93/44**

(51) Int. Cl.$^5$ : **C07C 2/08,** C07C 2/24, C07C 2/22

(21) Application number : **91302130.9**

(22) Date of filing : **13.03.91**

(54) **Process for oligomerizing olefins to prepare base stocks for synthetic lubricants.**

(30) Priority : **28.03.90 US 500631**
**14.05.90 US 522941**
**21.05.90 US 525807**
**30.04.90 US 516931**
**04.09.90 US 577385**
**30.04.90 US 516870**

(43) Date of publication of application : **02.10.91 Bulletin 91/40**

(45) Publication of the grant of the patent : **03.11.93 Bulletin 93/44**

(84) Designated Contracting States : **BE DE FR GB IT**

(56) References cited :
**GB-A- 1 489 646**
**US-A- 1 938 945**
**US-A- 4 531 014**

(73) Proprietor : **TEXACO CHEMICAL COMPANY**
**3040 Post Oak Boulevard**
**Houston, Texas 77056 (US)**

(72) Inventor : **Marquis, Edward Thomas**
**9004 Colinfield Drive**
**Austin, Texas 78758 (US)**
Inventor : **Sanderson, John Ronald**
**P O Box 587**
**Leander, Texas 78641 (US)**
Inventor : **Knifton, John Frederick**
**P O Box 15730**
**Austin, Texas 78726 (US)**

(74) Representative : **Brock, Peter William et al**
**URQUHART-DYKES & LORD 91 Wimpole Street**
**London W1M 8AH (GB)**

EP 0 449 453 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The invention relates to the preparation of synthetic lubricant base stocks, and more particularly to synthetic lubricant base stocks made by oligomerizing compositions comprising linear olefins by means of certain acidic montmorillonite clay catalysts.

Synthetic lubricants are prepared from man-made base stocks having uniform molecular structures, and consequently having well-defined properties that can be tailored to specific applications. Mineral oil base stocks, on the other hand, are prepared from crude oil and are complex mixtures of naturally occurring hydrocarbons. The greater uniformity of synthetic lubricants generally results in superior performance properties. For example, synthetic lubricants have excellent thermal stability. As automobile engines are reduced in size to save weight and fuel, they run at higher temperatures, and therefore require a more thermally-stable oil. Because lubricants made from synthetic base stocks have such properties as excellent oxidative/thermal stability, very low volatility, and good viscosity indices over a wide range of temperatures, they offer better lubrication and permit longer drain intervals, with less oil vaporization loss between oil changes.

Synthetic base stocks may be prepared by oligomerizing internal and alpha-olefin monomers to form a mixture of dimers, trimers, tetramers, and pentamers, with minimal amounts of higher oligomers. The unsaturated oligomer products are then hydrogenated to improve their oxidative stability. The resulting synthetic base stocks have uniform isoparaffinic hydrocarbon structures similar to high quality paraffinic mineral base stocks, but have the superior properties mentioned, due to their greater uniformity.

Synthetic base stocks are produced in a broad range of viscosity grades. It is common practice to classify the base stocks by their viscosities, measured in centistokes (cSt) at 100°C. Those base stocks with viscosities less than or equal to 4 cSt are commonly referred to as "low viscosity" base stocks, whereas base stocks having a viscosity in the range from 40 to 100 cSt are commonly referred to as "high viscosity" base stocks. Base stocks having a viscosity of 4 to 8 cSt are referred to as "medium viscosity" base stocks. Low viscosity base stocks generally are recommended for low temperature applications. Applications at higher temperatures, such as in motor oils, automatic transmission fluids, turbine lubricants, and other industrial lubricants, generally require higher viscosities, such as those provided by medium viscosity base stocks (i.e. 4 to 8 cSt grades). High viscosity base stocks are used in gear oils and as blending stocks.

The viscosity of the base stocks is determined by the length of the oligomer molecules formed during the oligomerization reaction. The degreeof oligomerization is affected by the catalyst and reaction conditions employed during the oligomerization reaction. The length of the carbon chain of the monomer starting material also has a direct influence on the properties of the oligomer products. Fluids prepared from short-chain monomers tend to have low pour points and moderately low viscosity indices, whereas fluids prepared from long-chain monomers tend to have moderately low pour points and higher viscosity indices. Oligomers prepared from long-chain monomers generally are more suitable than those prepared from shorter-chain monomers for use as medium viscosity synthetic lubricant base stocks.

One known approach to oligomerizing long-chain olefins to prepare synthetic lubricant base stocks, is to contact the olefin with boron trifluoride, together with a promotor, at a reaction temperature sufficient to effect oligomerization of the olefin; See, for example, US-A-4400565, -4420646, -4420647 and -4434308 and EP-A-0136377. Boron trifluoride gas ($BF_3$) is a pulmonary irritant, however, and breathing the gas, or fumes formed by hydration of the gas with atmospheric moisture, poses hazards preferably avoided. Moreover, the disposal/neutralization of $BF_3$ raises environmental concerns. Thus, a method for oligomerizing long-chain olefins using a non-hazardous, non-polluting catalyst would be a substantial improvement in the art.

Kuliev et al. attempted to prepare synthetic lubricants by oligomerizing long-chain ($C_9$-$C_{14}$) olefins using non-hazardous and non-polluting acidic clays, comprising sulphuric acid - and hydrochloric acid-activated bentonites from the Azerbaidzhan SSR; see Kuliev, Abasova, Gasanova, Kotlyarevskaya, and Valiev, "Preparation of High-Viscosity Synthetic Lubricants Using an Aluminosilicate Catalyst," Institute of Petrochemical Processes of the Academy of Sciences of the Azerbaidzhan SSR, Azer. Neft. Khoz., 1983, No. 4, pages 40-43. Kuliev et al. concluded, however, that "it was not possible to prepare viscous or high-viscosity oils by olefin polymerization over an aluminosilicate catalyst" and that "hydrogen redistribution reactions predominate with formation of aromatic hydrocarbon, coke, and paraffinic hydrocarbon." US-A-453 1014 discloses the use of Wyoming bentonite to oligomerize shorter-chain olefins, but this process does not make it possible to obtain a product high in dimer, trimer and tetramer, and low in disproportionation products.

We have surprisingly discovered that it is possible to prepare synthetic lubricant base stocks in good yield by oligomerizing long-chain olefins using certain acidic montmorillonite clay catalysts. We have found that a high conversion of long-chain olefin to dimer, trimer, and tetramer may be obtained, with formation of very little concomitant hydrogen redistribution by-product, by using certain acidic calcium montmorillonite clays.

One embodiment of this invention relates to a process for the preparation of oligomers or co-oligomers of

linear olefins containing from 10 to 24 carbon atoms in which an olefin composition comprising said olefins is contacted with a catalyst. The catalyst is an acidic calcium montmorillonite clay having a moisture content up to 20 wt.%, a residual acidity of up to 30 mg KOH/g, and a surface area of 300 $M^2/g$ or greater.

The linear olefins may comprise alpha-olefins, internal olefins, or a mixture thereof. Optionally, the linear olefins may be employed in admixture with up to 20 weight % of propylene, or of 1,3-diisopropenyl benzene or alpha-methylstyrene. They may also be employed in admixture with vinylidene olefins having 10 to 24 carbon atoms, eg. in amount of 5 to 40 weight %.

In a preferred embodiment, at least 0.01 wt% of a Lewis Acid selected from aluminium nitrate, zinc chloride, and nickel chloride may be deposited on the day.

According to another preferred embodiment, the oligomerized product is hydrogenated.

The olefin monomer feed stocks used in the present invention may be selected from (1) alpha-olefins having the formula $R''CH=CH_2$, where R" is alkyl having 8 to 22 carbon atoms, and (2) internal olefins having the formula RCH=CHR', where R and R' are the same or different alkyl having 1 to 20 carbon atoms, provided that the total number of carbon atoms in any one olefin is from 10 to 24, inclusive. A preferred range for the total number of carbon atoms in any one olefin molecule is 12 to 18, inclusive, with an especially preferred range being 13 to 16, inclusive. Mixtures of internal and alpha-olefins may be used, as well as mixtures of olefins having different numbers of carbon atoms, provided that the total number of carbon atoms in any one olefin is from 10 to 24, inclusive. The alpha and internal-olefins to be oligomerized in this invention may be obtained by processes well-known to those skilled in the art and are commercially available.

According to one embodiment of the invention, it has been discovered that certain properties of these synthetic lubricant base stocks are improved when the olefin feed comprises a mixture of alpha and internal-olefin having up to 50 wt.% of internal-olefin. When oligomers produced in this manner are hydrogenated, they yield synthetic lubricant base stocks having higher viscosity indices. A higher viscosity index indicates that the synthetic lubricant will be less susceptible to a change in viscosity when subjected to a change in temperature. This is a desirable characteristic for most lubricating applications. In addition, these synthetic lubricants have a lower pour point, another desirable feature.

The oligomerization reaction may be carried out in either a stirred slurry reactor or in a fixed bed continuous flow reactor. In a continuous reaction, up to 50 wt.% of the olefin feed may be made to comprise internal-olefin by either or both of two methods. According to the first method, separate feedstreams of internal and alpha-olefins may be mixed in the desired weight ratio before or upon entering the reactor containing the clay catalyst bed. Alternatively, or in addition, the second method provides that internal olefin may be introduced to the catalyst bed in a recycle stream. When alpha-olefin in the feedstream passes through the clay catalyst in the reactor, a portion of the olefin remains un-oligomerized. Most of the olefin remaining in monomer form will have isomerized to internal olefin. Thus, the product of the oligomerization reaction has three components: oligomer; internal-olefin monomer; and alpha-olefin monomer. The alpha and internal-olefins may be stripped from the oligomer and recycled to the catalyst bed for oligomerization. The monomer stripping step should be conducted under mild conditions. Distillation at temperatures exceeding 210°C may cause the oligomers to break down in some fashion and come off as volatiles. Preferably, therefore, the reboiler or pot temperautre should be kept at or under 180°C when stripping out the monomer. Procedures known by those skilled in the art to be alternatives to fractional distillation also may be employed to separate the monomers from oligomers.

In summary, internal olefin may be introduced to the catalyst bed directly as a feedstream component and/or as a recycled isomer of the alpha-olefin feed. Additionally, when internal-olefin is introduced as a feedstream component, any un-oligomerized internal-olefin also may be recycled to the catalyst bed. When either or both methods are employed, no more than 50 wt.% of the olefin present in the catalyst bed at any one time should comprise internal-olefin.

In a batch reaction system, internal-olefin should comprise up to 50 wt.% of the olefin starting material. The internal-olefin component may be obtained by processes known to those skilled in the art, purchased commercially, and/or may be distilled from the product of a prior oligomerization reaction and recycled by inclusion in a subsequent batch oligomerization reaction.

According to another embodiment, it has surprisingly been found that synthetic lubricant base stocks with a lower pour point and an improved viscosity index may be obtained when the oligomers are prepared by co-oligomerizing a mixture of up to 20 wt.% as propylene and more than 80 wt.% of the $C_{10}$-$C_{24}$ olefin, preferably alpha-olefin, in the presence of the clay catalysts. Moreover, incorporating the propylene lowers the cost of producing the base stocks, by replacing a portion of the more expensive long-chain olefin feed with propylene.

Preferably, the mixture of $C_{10}$-$C_{24}$ olefin and propylene contains from 2 to 10 wt.% of propylene. When oligomers produced in this manner are hydrogenated, they yield synthetic lubricant base stocks having a lower pour point. Additionally, when the propylene comprises no more than 15 wt. % of the $C_{10}$-$C_{24}$ olefin/propylene mixture, the resulting synthetic lubricants have a higher viscosity index.

According to a further embodiment of the invention, synthetic lubricant base stocks with a lower pour point and a higher viscosity may be obtained when the oligomers are prepared by co-oligomerizing a mixture of up to 20 wt.% of 1,3-di-isopropenyl benzene or alpha-methylstyrene and more than 80 wt.% of $C_{10}$-$C_{24}$ olefin, preferably alpha-olefin, in the presence of the clay catalysts.

Preferably, the mixture of $C_{10}$-$C_{24}$ olefin and 1,3-di-isopropenyl benzene or alpha-methylstyrene contains from 5 to 15 wt.% of 1,3-di-isopropenyl benzene or alpha-methylstyrene. It is most preferred that the mixture contains about 10 wt.% of 1,3-di-isopropenyl benzene or alpha-methylstyrene. When oligomers produced in this manner are hydrogenated, they yield synthetic lubricant base stocks having a lower pour point.

Additionally, when the 1,3-di-isopropenyl benzene comprises more than 5 wt.% of the $C_{10}$-$C_{24}$ olefin/1,3-di-isopropenyl benzene mixture, the resulting synthetic lubricants have a higher viscosity.

According to a further embodiment, it is possible to use the clay catalysts for co-oligomerizing a mixture of (1) a long-chain ($C_{10}$-$C_{24}$) vinylidene olefin and (2) the above-mentioned straight chain $C_{10}$-$C_{24}$ alpha-olefins and/or internal-olefins. US-A-4214111 discloses co-polymerizing higher alpha-olefins with a vinylidene olefin, but, the vinylidene olefin is a shorter-chain olefin, (isobutylene or di-isobutylene), and the catalyst employed is an aluminium halide catalyst, which may be corrosive. The process according to the present invention achieves a high conversion of olefin to oligomers incorporating higher molecular wieght vinylidenes using a more easily handled catalyst.

It has been further discovered that the pour point of the synthetic lubricant base stocks is improved when the olefin feed comprises a mixture of $C_{10}$-$C_{24}$ vinylidene olefin and $C_{10}$-$C_{24}$ alpha- and/or internal-olefin. Preferably, from 5 to 40 wt.% of the mixture of starting materials comprises vinylidene olefin. More preferably, from 15 to 30 wt.% of the mixture of starting materials comprises vinylidene olefin. It is especially preferred that about 20 wt.% of the starting materials is vinylidene olefin.

The vinylidene olefins have the formula

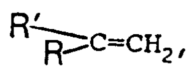

where R and R' are the same or different alkyl radicals of 1 to 21 carbon atoms, provided that (as with the straight chains olefins) the total number of carbon atoms in any one olefin is from 10 to 24, inclusive, preferably 14 to 18, and more preferably 14 to 16 carbon atoms. Mixtures containing the vinylidene olefins and both internal- and alpha- olefins may be used, as well as mixtures containing vinylidene olefins and either internal- or alpha-olefins. The greatest improvement in pour point, over base stocks prepared without the inclusion of vinylidene olefin, is observed when base stocks prepared from alpha-olefins alone are compared with base stocks prepared from mixtures which include vinylidene olefins. Only a small improvement is observed over base stocks which include a substantial percentage of internal olefin.

The oligomerization reaction may be represented by the following general equation:

$$nC_mH_{2m} \xrightarrow{\text{catalyst}} C_{mn}H_{2mn}$$

where n represents moles of monomer and m represents the number of carbon atoms in the monomer. Thus, the oligomerization of 1-decene may be represented as follows:

$$nC_{10}H_{20} \xrightarrow{\text{catalyst}} C_{10n}H_{20n}$$

The co-oligomerization of straight chain with vinylidene olefins will follow a corresponding course.

The reaction occurs sequentially. Initally, olefin monomer reacts with olefin monomer to form dimers. The dimers that are formed then react with additional olefin monomer to form trimers, and so on. This results in an oligomer product distribution that varies with reaction time. As the reaction time increases, the olefin monomer conversion increases, and the selectivities for the heavier oligomers increase. Generally, each resulting oligomer contains one double bond.

Similarly, co-oligomerization of propylene and 1-decene may be represented as follows:

$$nC_{10}H_{20} + XC_3H_6 \xrightarrow{\text{catalyst}} C_{(n10+3x)}H_{(20n+6x)}$$

where n represents moles of $C_{10}$-$C_{24}$ olefin and x represents moles of propylene.

The co-oligomerization of 1,3-di-isopropenyl benzene and a $C_{10}$-$C_{24}$ olefin, such as 1-decene, may result in several reactions. For example, one propenyl group of the 1,3-di-isopropenyl benzene may react with decene, as represented by the following equation:

$$nC_{10}H_{20} + XC_6H_4[C(CH_3) = CH_2]_2 \xrightarrow{\text{catalyst}} C_{(n10+3x)}H_{(20n+5x)}$$

$$[C_6H_4C(CH_3) = CH_2]_x$$

where n represents moles of $C_{10}$-$C_{24}$ olefin and x represents moles of 1,3-di-isopropenyl benzene. Additionally, both propenyl groups may react with decene. One or both propenyl groups also may react with other 1,3-di-isopropenyl benzene molecules. Finally, 1,3-di-isopropenyl benzene is a reactive aromatic, and may undergo aromatic substitution. The substituted aromatic may then react with decene or 1,3-di-isopropenyl benzene.

A similar course is followed when alpha-methylstyrene is co-oligomerized with the $C_{10}$-$C_{24}$ olefin.

The catalysts used to effect the oligomerization reaction according to invention are certain silica-alumina clays, also called aluminosilicates. Silica-alumina clays primarily are composed of silicon, aluminium, and oxygen, with minor amounts of magnesium and iron in some instances. Variations in the ratios of these constituents, and in their crystal lattice configurations, result in some fifty separate clays, each with its own characteristic properties.

Smectite clays are one class of silica-alumina clays. Smectite clays have a small particle size and unusual intercalation properties which afford them a high surface area. Smectites comprise layered sheets of octahedral sites between sheets of tetrahedral sites, where the distance between the layers can be adjusted by swelling, using an appropriate solvent. Three-layered sheet-type smectites include montmorillonites. The montmorillonite structure may be represented by the following formula:

$$\overset{n+}{M_{x/n}} \cdot yH_2O(Al_{4-x}Mg_x)\ (Si_8)O_{20}(OH)_4$$

where M represents the interlamellar (balancing) cations, normally sodium or lithium; and x, y and n are numbers.

Montmorillonite clays may be acid-activated by such mineral acids as sulphuric acid and hydrochloric acid. Mineral acids activate montmorillonites by attacking and solubilizing structural cations in the octahedral layers. This opens up the clay structure and increases surface area. These acid-treated clays act as strong Bronsted acids. We have discovered that certain acid- treated montmorillonite clay catalysts are particularly effective for preparing synthetic lubricant base stocks in good yield by oligomerizing long-chain olefins. These clays are acidic calcium montmorillonite clays having a moisture content up to 20 wt.%, a residual acidity from 3 to 30 mg KOH/g (when titrated to a phenolphthalein end point), and a surface area of 300 $M^2/g$ or more. Illustrative examples include Filtrol grade 24, having a moisutre content of 12 wt.%, a residual acidity of 8.5 mg KOH/g, and a surface area of 425 $M^2/g$; Filtrol grade 124, having a moisture content of 2 wt.%, a residual acidity of 7.0 mg KOH/g, and a surface area of 400 $M^2/g$; Filtrol grade 13, having a moisture content of 16 wt.%, a residual acidity of 15 mg KOH/g, and a surface area of 300 $M^2/g$; Filtrol grade 113, having a moisture content of 4 wt.%, a residual acidity of 10 mg KOH/g, and a surface area of 300 $M_2/g$; and Filtrol grade 224, having virtually no moisture, and having a residual acidity of 3.0 mg KOH/g, and a surface area of 350 $M^2/g$.

Preferably, the catalyst is activated by heat treatment before carrying out the oligomerization reaction. We have found that heat treatment of the catalyst before the oligomerization reaction causes the catalyst to be more active and produce a higher olefin conversion. Additionally, clays heat treated in this manner are more stable, remaining active over longer periods during the oligomerization reaction. The clays may be heat treated at temperatures of 50 to 400°C, with or without the use of a vacuum. A more preferred temperature range is 50 to 300°C. Optionally, an inert gas may also be used during heat treatment. Preferably, the clay should be heat treated under conditions, and for a length of time, which will reduce the water content of the clay to 1 wt.% or less.

The oligomerization reaction may be carried out in either a stirred slurry reactor or in a fixed bed continuous flow reactor. The catalyst concentration should be sufficient to provide the desired catalytic effect. The temperatures at which the oligomerization may be performed are generally between 50 and 300°C, with the preferred range being from 150 to 180°C. The reaction may be run at pressures from ambient to 7 MPa (0 to 1000 psig).

According to another embodiment of the present invention, we have surprisingly discovered that an even higher conversion of olefin into oligomer may be obtained when the acidic calcium montmorillonite clays are treated with a Lewis acid such as aluminium nitrate before they are used as a catalyst. Additionally, the resulting oligomer products contain a greater percentage of trimer and higher oligomers.

In the preferred form of this embodiment of the present invention, the clay is treated with aluminium nitrate before running the reaction. The clay can be added to a solution of 1 to 20 wt.%, preferably 10 wt.% of aluminium nitrate in water. The ratio of clay to aluminium nitrate solution should be sufficient to provide a washed and dried catalyst having a concentration of aluminium nitrate at least sufficient to give the Friedel-Crafts effect, preferably 0.01 to 10 wt.% of aluminium nitrate, but not so much that it inhibits the acid sites of the clay. The clay should remain in the aluminium nitrate solution under agitation for a period sufficient to meet these requirements.

We have also found that advantageous results may be obtained when certain other Lewis Acid moderators are employed in the place of aluminium nitrate. For example, acidic montmorillonite clays treated with either nickel chloride or zinc chloride were found to yield a higher conversion of monomer into oligomer, and to produce an oligomer product having a better dimer/trimer ratio, than untreated acidic clays. Neither the nickel chloride-treated clay, nor the zinc chloride-treated clay, was capable, however, of achieving the conversion and dimer/trimer ratio obtained with the aluminium nitrate-treated clay, the preferred Lewis Acid treatment in the present invention. Other Lewis Acids may provide comparable results. However, clays treated with either of two other Lewis Acids, zirconium chloride and aluminium chloride, were found to produce a lower conversion of monomer to oligomer and/or a poorer dimer/trimer ratio than untreated clays.

Preferably, the aluminium nitrated-treated clay catalyst is heat-treated before running the reaction. The conditions used are generally the same as those disclosed above for the clays that have not been treated with Lewis acids.

Following the oligomerization reaction, the unsaturated oligomers may be hydrogenated to improve their thermal stability and to guard against oxidative degradation during their use as lubricants. The hydrogenation reaction for 1-decene oligomers may be represented as follows:

$$C_{10n}H_{20n} + H_2 \xrightarrow{\text{catalyst}} C_{10n}H_{(20n+2)}$$

where n represents moles of monomer used to form the oligomer. Hydrogenation of propylene/1-decene co-oligomers may be represented as follows:

$$C_{(10n+3x)}H_{(20n+6x)} + H_2 \xrightarrow{\text{catalyst}} C_{(10n3x)}H_{(20n+6x+2)}$$

where n and x represent moles of long-chain alpha-olefin and propylene, respectively, used to form the co-oligomer.

Hydrogenation of 1,3-di-isopropenyl benzene/1-decene co-oligomers may be represented as follows:

$$C_{(10n+3x}H_{(20n+5x)}[C_6H_4C(CH_3)=CH_2]_x - H_2 \xrightarrow{\text{catalyst}} C_{(10n+3x)}H_{(20n+5x+2)}[C_6H_4C(CH_3)=CH_2]_x$$

where n and x represent moles of long-chain alpha-olefin and 1,3-di-isopropenyl benzene, respectively, used to form the co-oligomer.

Hydrogenation processes known to those skilled in the art may be used to hydrogenate the oligomers. A number of metal catalysts are suitable for promoting the hydrogenation reaction, including nickel, platinum, palladium, copper, and Raney nickel. These metals may be supported on a variety of porous materials such as kieselguhr, alumina, or charcoal. A particularly preferred catalyst for this hydrogenation is a nickel-copper-chromia catalyst described in US-A-3152998. Other known hydrogenation procedures are described in US-A-4045508, -4013736, -3997622 and -3997621.

If, in a continuous reaction, the recycle stream is not employed, it is desirable to strip any un-oligomerized monomer from the oligomer product.

While it is known to include a distillation step after the hydrogenation procedure to obtain products having various viscosities at 100°C, it is preferred in the method of the present invention that no further distillation (beyond monomer flashing) should be conducted. In other words, the monomer-stripped, hydrogenated bottoms are the desired synthetic lubricant components. Thus, the method of this invention does not require the costly, customary distillation step, yet, surprisingly, produces a synthetic lubricant component that has excellent properties and that performs in a superior fashion. In some instances, however, one skilled in the art may find subsequent distillation useful in the practice of this invention.

The monomer stripping step should be conducted under mild conditions. Distillation at temperatures exceeding 210°C may cause the oligomers to break down in some fashion and come off as volatiles. Preferably, therefore, the reboiler or pot temperature should be kept at or under 180°C when stripping out the monomer.

The invention will be further illustrated by the following Examples, which are given by way of illustration.

## EXAMPLE 1

The following demonstrates the use of the present invention in batch reaction systems:

### Batch-Flask

Olefin and clay catalyst were charged to a flask equipped with a stirrer, thermometer, heating mantle, and a water-cooled condenser ($N_2$ purge). The mixture was vigorously stirred and heated at a desired temperature for the desired time. The mixture was then cooled to ambient temperature and filtered with suction. The liquid was analyzed by liquid chromatography. The results are detailed in Table I.

### Batch-Autoclave

Olefin and clay catalyst were charged to an autoclave. The autoclave was sealed and heated to the desired temperature for the desired time. The mixture was then cooled to ambient temperature and filtered with suction. The liquid was then analyzed by liquid chromatography. The results are shown in Table I.

### Hydrogenation of Oligomer

An autoclave was charged with oligomer prepared in Batch No. 6 of Table I and finely powdered nickel catalyst. The autoclave was flushed with hydrogen and then pressurized to 7 MPa with hydrogen. The mixture was heated to 200°C and stirred at this temperautre for 4 hours. The mixture was then repressurized with hydrogen to 13.89 MPa as needed. The mixture was then cooled to ambient temperature, the catalyst was filtered, and the monomer was removed. Typical results are shown in Table II.

TABLE 1

| Btch No. | Olefin(s) (by carbon number) | (g) | Harshaw/ Filtrol | (g) | Reactor | Time/Temp (H)/(°C) | Con. (%) | Results from LC M(%) | D(%) | T+(%) | D/T+ Ratio |
|---|---|---|---|---|---|---|---|---|---|---|---|
| 1 | C-14A | 101 | H/F Clay 13 | 25 | Flask | 3/150 | 81.9 | 18.1 | 49.1 | 32.8 | 1.50 |
| 2 | C-14A | 100 | H/F Clay 24 | 25 | Flask | 4/150 | 92.4 | 7.6 | 43.9 | 48.4 | 0.91 |
| 3 | C-14A | 100 | H/F Clay 13 | 10 | Clave | 4/300 | 78.9 | 21.1 | 55.3 | 23.4 | 2.36 |
| 4 | C-14A | 100 | H/F Clay 24 | 10 | Clave | 4/300 | 57.6 | 42.4 | 46.4 | 11.2 | 4.14 |
| 5 | C-14A | 100 | 85% $H_3PO_4$ | 10 | Clave | 4/300 | 15.6 | 84.4 | 7.87 | 7.69 | 1.02 |
| 6 | C-14A | 350 | H/F Clay 24 | 50 | Flask | 4/150 | 80.0 | 20.0 | 49.0 | 31.0 | 1.58 |
| 7 | C-131,141(40% 60%) | 350 | H/F Clay 24 | 50 | Flask | 4/150 | 63.5 | 36.5 | 45.7 | 17.7 | 2.58 |
| 8 | C-131,141C-14A | 250 100 | H/F Clay 24 | 50 | Flask | 4/150 | 67.1 | 32.9 | 48.7 | 18.4 | 2.65 |
| 9 | C-131,141(40%, 60%) | 350 | H/F Clay 24 | 50 | Flask | 4/180 | 78.0 | 22.0 | 51.1 | 26.9 | 1.90 |
| 10 | C-151, 161 | 350 | H/F Clay 24 | 50 | Flask | 4/150 | 45.6 | 54.5 | 33.7 | 11.8 | 2.86 |
| 11 | C-151, 161 | 350 | H/F Clay 24 | 50 | Flask | 16/120 | 8.4 | 91.6 | 8.4 | -0 | - |
| 12 | C-14A, 16A (63%,36%) | 350 | H/F Clay 24 | 50 | Flask | 3/150 | 78.6 | 21.4 | 48.6 | 30.0 | 1.62 |
| 13 | C-14A,16A(63% 36%) | 400 | H/F Clay 24 | 20 | Flask | 6/180 | 78.1 | 21.9 | 48.9 | 29.3 | 1.67 |
| 14 | C-14A,16A(63% 36%) | 400 | H/F Clay 24 | 10 | Flask | 7/180 | 63.5 | 36.5 | 48.7 | 24.8 | 1.96 |
| 15 | C-14A,16A(63% 36%) | 400 | H/F Clay 24 | 40 | Flask | 6/150 | 78.9 | 21.1 | 47.6 | 31.4 | 1.52 |

Con. = Conversion; M = Monomer; D = Dimer, T + = Trimer + Teramer + etc.
A = Alpha; I = Internal

TABLE II

PROPERTIES OF HYDROGENATED OLIGOMER FROM BATCH NO. 6 (TABLE I)

| | |
|---|---|
| Monomer | 1.69 % |
| Dimer | 58.5 % |
| Trimer | 29.6 % |
| Tetramer | 9.53 % |
| Pentamer | 0.76 % |
| Pour Point (°C) | -34.5 |
| Viscosity | 39.4 cSt (-4°C) |
| | 24.4 cSt (38°C) |
| | 4.69 cSt (99°C) |
| Viscosity Index | 125 |
| % Remaining by Thermogravimetric Analysis | |
| (233°C) | 90.5 % |
| (250°C) | 83.5 % |

EXAMPLE 2

The following tables demonstrate the use of the present invention in continuous reaction systems. The 100 cc reactor (see Table III) was a 74 x 1.6cm stainless steel tube. The catalyst bed was about 100 cc. Liquid feed was pumped through the bottom of the reactor with a Ruska pump. A Uni-flow valve and a Foxboro controller were used to regulate pressure. The reactor was electrically heated. The 300 cc reactor (see Table IV) was a 74 x 2.3cm stainless steel tube. The catalyst bed was about 300 cc. Other equipment was the same as described in connection with the 100 cc reactor.

The olefin was NEODENE 1518 IO of Shell Chemical Co., an internal olefin having the following constitution:-

| | |
|---|---|
| $C_{14}$ and lower | 1.8 % |
| $C_{15}$ | 25.3 % |
| $C_{16}$ | 26.3 % |
| $C_{17}$ | 24.2 % |
| $C_{18}$ | 18.5 % |
| $C_{19}$ | 3.9 % |

TABLE III

| Sample No. | Liquid Flow (CC) | Temp (C) | Reactor Press. (psig MPa) | Time Since Prior Sample (H) | C(%) | M(%) | D(%) | T+(%) | D/T + Ratio |
|---|---|---|---|---|---|---|---|---|---|
| 1 | 18 | 150 | 200/1.48 | 4 | 75.5 | 24.5 | 43.9 | 31.6 | 1.39 |
| 2 | 18 | 156 | 200/1.48 | 6 | 82.4 | 17.6 | 41.6 | 40.8 | 1.02 |
| 3 | 18 | 169 | 300/2.17 | 17 | 80.5 | 19.5 | 42.4 | 38.1 | 1.11 |
| 4 | 18 | 200 | 300/2.17 | 6 | 73.2 | 26.8 | 44.5 | 28.7 | 1.56 |
| 5 | 20 | 200 | 300/2.17 | 16 | 77.1 | 22.9 | 52.0 | 25.1 | 2.07 |
| 6 | 20 | 150 | 300/2.17 | 6 | 69.4 | 30.6 | 46.5 | 22.9 | 2.03 |
| 7 | 20 | 149 | 300/2.17 | 15 | 64.1 | 35.9 | 46.5 | 12.6 | 2.64 |
| 8 | 20 | 149 | 300/2.17 | 4 | 61.9 | 38.1 | 42.3 | 14.6 | 3.28 |
| 9 | 20 | 155 | 300/2.17 | 4 | 48.1 | 51.9 | 32.5 | 15.6 | 2.08 |
| 10 | 20 | 156 | 300/2.17 | 4 | 54.0 | 58.4 | 35.0 | 19.0 | 1.84 |
| 11 | 20 | 156 | 300/2.17 | 15 | 58.4 | 41.6 | 39.8 | 18.7 | 2.13 |
| 12 | 20 | 155 | 300/2.17 | 9 | 59.2 | 40.8 | 40.2 | 19.0 | 2.12 |
| 13 | 20 | 155 | 300/2.17 | 14 | 62.8 | 37.2 | 41.8 | 21.0 | 1.99 |
| 14 | 20 | 155 | 300/2.17 | 7 | 65.8 | 34.2 | 41.8 | 24.0 | 1.24 |
| 15 | 20 | 155 | 300/2.17 | 15 | 67.5 | 32.5 | 42.4 | 25.1 | 1.69 |
| 16 | 20 | 155 | 300/2.17 | 4 | 65.0 | 35.0 | 43.9 | 21.2 | 2.08 |
| 17 | 20 | 153 | 300/2.17 | 15 | 68.8 | 31.2 | 45.7 | 34.1 | 1.98 |

TABLE III

| Sample No. | Liquid Flow (CC) | Temp (C) | Reactor Press. (psig MPa) | Time Since Prior Sample (H) | C(%) | M(%) | D(%) | T+(%) | D/T + Ratio |
|---|---|---|---|---|---|---|---|---|---|
| 18 | 20 | 156 | 300/2.17 | 9 | 73.7 | 26.3 | 48.3 | 25.4 | 1.90 |
| 19 | 20 | 156 | 300/2.17 | 15 | 77.2 | 22.8 | 47.4 | 29.8 | 1.59 |
| 20 | 20 | 156 | 300/2.17 | 15 | 77.2 | 22.8 | 47.4 | 29.8 | 1.59 |
| 21 | 20 | 156 | 300/2.17 | 16 | 72.3 | 22.7 | 49.3 | 28.0 | 1.76 |
| 22 | 20 | 156 | 300/2.17 | 24 | 74.4 | 25.6 | 48.6 | 25.8 | 1.89 |
| 23 | 20 | 156 | 300/2.17 | 8 | 72.4 | 27.6 | 49.6 | 22.8 | 2.18 |
| 24 | 20 | 156 | 300/2.17 | 16 | 71.1 | 8.9 | 48.0 | 23.2 | 2.07 |
| 25 | 20 | 156 | 300/2.17 | 24 | 68.1 | 31.9 | 32.3 | 20.7 | 2.28 |
| 26 | 20 | 158 | 300/2.17 | 9 | 66.4 | 33.6 | 46.8 | 19.6 | 2.39 |
| 27 | 20 | 156 | 300/2.17 | 24 | 66.5 | 33.3 | 46.2 | 20.3 | 2.27 |
| 28 | 20 | 156 | 300/2.17 | 15 | 64.1 | 35.9 | 46.4 | 12.9 | 2.62 |
| 29 | 20 | 156 | 300/2.17 | 9 | 64.1 | 39.9 | 44.1 | 20.0 | 2.21 |
| 30 | 20 | 156 | 300/2.17 | 15 | 62.4 | 37.6 | 48.1 | 18.4 | 2.40 |
| 31 | 20 | 156 | 300/2.17 | 9 | - | - | - | - | - |
| 32 | 20 | 156 | 300/2.17 | 15 | - | - | - | - | - |
| 33 | 20 | 155 | 300/2.17 | 9 | 57.9 | 42.1 | 41.8 | 16.7 | 2.46 |
| 34 | 20 | 154 | 154/1.16 | 15 | 56.6 | 43.4 | 42.2 | 16.4 | 2.93 |

Distillation of Oligomers Prepared from C-12 Alpha-Olefin

Fractions from a $C_{12}$ run described in Table III were conbined to give 1741 grams of product, which had the following analysis by liquid chromatography:

| | |
|---|---|
| Monomer | 27.8 % |
| Dimer | 48.0 % |
| Trimer | 19.3 % |
| Tetramer | 4.91% |

This material was vacuum distilled through a 25cm Goodloe packed column until a pot temperature of 236°C was reached. Four fractions were collected. The fourth fraction, boiling point 165 - 170°C, at 1.1KPa, was 93.4% $C_{12}$ dimer. The pot residue was free of dimer and monomer. The reduced stripped dimer had the following properties:

| | |
|---|---|
| Viscosity 99°C | 2.50 cSt |
| Viscosity Index | 78.5 |
| Pour Point °C | <-45.5 |
| % Remaining by TGA | |
| 233°C | 30% |
| 250°C | 1% |

The reduced pot residue had the following properties:

| | |
|---|---|
| Viscosity (99°C) | 8.40 cSt |
| Viscosity Index | 128 |
| Pour Point (°C) | -29 |
| % Remaining by TGA | |
| 233°C | 99% |
| 250°C | 98% |

TABLE IV

| Sample No. | Liquid Flow (CC) | Temp (C) | Reactor Press. (psig MPa) | Time Since Prior Sample (H) | C(%) | M(%) | D(%) | T+(%) | D/T + Ratio |
|---|---|---|---|---|---|---|---|---|---|
| 1 | 48 | 150/155 | 300/2.17 | 3 | 5.4 | 94.5 | 5.1 | 0.4 | 12.8 |
| 2 | 48 | 151/158 | 300/3.17 | 15 | 32.4 | 67.6 | 24.0 | 8.4 | 2.86 |
| 3 | 48 | 151/158 | 300/2.17 | 4 | 64.3 | 35.7 | 39.3 | 25.0 | 1.57 |
| 4 | 48 | 151/157 | 300/2.17 | 5 | 59.1 | 40.9 | 44.4 | 14.7 | 3.02 |
| 5 | 48 | 152/157 | 300/2.17 | 15 | 58.6 | 41.4 | 44.6 | 14.1 | 3.16 |
| 6 | 48 | 148/153 | 300/2.17 | 25 | 64.4 | 35.6 | 47.6 | 16.7 | 2.85 |
| 7 | 48 | 149/155 | 300/2.17 | 8 | 68.1 | 32.0 | 43.9 | 19.4 | 2.26 |
| 8 | 48 | 146/154 | 300/2.17 | 15 | 65.2 | 34.8 | 48.3 | 16.9 | 2.86 |
| 9 | 48 | 148/152 | 300/2.17 | 9 | 68.6 | 31.5 | 45.1 | 23.4 | 1.93 |
| 10 | 48 | 143/152 | 300/2.17 | 4 | 62.4 | 37.6 | 42.7 | 19.8 | 2.16 |
| 11 | 48 | 143/152 | 300/2.17 | 19 | 62.0 | 38.0 | 43.1 | 19.0 | 2.27 |
| 12 | 48 | 141/150 | 300/2.17 | 5 | 61.5 | 38.5 | 43.6 | 17.9 | 2.44 |
| 13 | 48 | 141/150 | 300/2.17 | 4 | 63.1 | 36.9 | 43.9 | 19.2 | 2.29 |
| 14 | 48 | 143/152 | 300/2.17 | 15 | 62.7 | 37.3 | 43.5 | 19.1 | 2.28 |
| 15 | 48 | 145/153 | 300/2.17 | 9 | 60.1 | 39.9 | 46.7 | 23.4 | 2.00 |
| 16 | 48 | 147/157 | 300/2.17 | 16 | 59.2 | 40.8 | 45.2 | 14.0 | 3.23 |
| 17 | 48 | 147/156 | 300/2.17 | 8 | 63.4 | 36.6 | 44.6 | 18.8 | 2.37 |
| 18 | 48 | 147/156 | 300/2.17 | 15 | 60.2 | 39.9 | 46.6 | 13.5 | 3.45 |

TABLE IV

| Sample No. | Liquid Flow (CC) | Temp (C) | Reactor Press. (psig MPa) | Time Since Prior Sample (H) | C(%) | M(%) | D(%) | T+(%) | D/T + Ratio |
|---|---|---|---|---|---|---|---|---|---|
| 19 | 48 | 151/159 | 300/2.17 | 9 | 59.2 | 40.8 | 46.1 | 13.1 | 3.52 |
| 20 | 48 | 151/159 | 300/2.17 | 15 | 55.4 | 44.6 | 44.1 | 11.4 | 3.89 |
| 21 | 48 | 151/159 | 300/2.17 | 3 | 56.3 | 43.7 | 39.9 | 16.2 | 2.46 |
| 22 | 48 | 141/155 | 300/2.17 | 15 | 47.7 | 52.3 | 38.7 | 8.96 | 4.32 |
| 23 | 48 | 145/155 | 300/2.17 | 9 | 48.0 | 52.0 | 39.0 | 9.00 | 4.33 |
| 24 | 48 | 145/155 | 300/2.17 | 15 | 43.9 | 56.1 | 35.2 | 8.69 | 4.05 |
| 25 | 48 | 147/156 | 300/2.17 | 9 | 39.7 | 60.4 | 32.5 | 2.13 | 4.56 |
| 26 | 48 | 147/156 | 300/2.17 | 15 | 41.0 | 59.0 | 33.3 | 2.32 | 4.31 |
| 27 | 48 | 139/155 | 300/2.17 | 4 | 25.6 | 24.4 | 20.7 | 4.93 | 4.20 |
| 28 | 48 | 145/155 | 300/2.17 | 15 | 39.5 | 60.5 | 31.0 | 8.43 | 3.68 |
| 29 | 48 | 146/155 | 300/2.17 | 4 | 35.9 | 64.1 | 30.2 | 5.74 | 5.26 |
| 30 | 48 | 146/155 | 300/2.17 | 17 | 32.2 | 67.8 | 26.8 | 5.43 | 4.94 |
| 31 | 48 | 146/155 | 300/2.17 | 9 | 29.9 | 70.1 | 24.3 | 5.64 | 4.31 |
| 32 | 56 | 146/155 | 300/2.17 | 15 | 28.0 | 72.0 | 23.5 | 4.59 | 5.12 |
| 33 | 50 | 148/158 | 300/2.17 | 9 | 26.0 | 74.0 | 21.8 | 4.22 | 5.17 |
| 34 | 50 | 148/158 | 300/2.17 | 15 | 27.6 | 78.4 | 23.1 | 4.55 | 5.08 |
| 35 | 50 | 148/158 | 300/2.17 | 9 | 28.8 | 71.2 | 23.7 | 5.09 | 4.66 |

Distillation of Oligomers Prepared from Neodene 1518 IO

Samples 2, 5, 6, 7, and 8 from Table IV were combined to yield a total of 2600 grams of material, which had the following analysis by liquid chromatography:

| | |
|---|---|
| Monomer | 37.8 % |
| Drimer | 40.2 % |
| Trimer | 17.8 % |
| Tetramer | 4.08% |

The material was placed in a five-liter flask and distilled through a 25cm Goodloe packed column until a pot temperature of 270°C was reached (1.1 kP). The pot residue (976 grams) was reduced with hydrogen and a nickel catalyst (5 wt.% catalyst, 200°C, 4.0 hours 13.9 MPa hydrogen). The material obtained after filtration had the following properties:

| | |
|---|---|
| Viscosity (99°C) | 8.40 cSt |
| Viscosity Index | 128 |
| % Remaining by TGA | |
| 233°C | 99 % |
| 250°C | 98 % |

EXAMPLE 3

Ths example demonstrates the superior results obtained when the clay catalysts are heat treated before oligomerizing the olefin.

Filtrol Clay-124 (200 grams) was placed in a vacuum and heat treated overnight for 20 hours at approximately 160°C and a pressure of approximately 2.67 kpa. Before being heat treated, the clay had a moisture content of 7.11 wt.%. After being heat treated, the moisture content was 1.03 wt.%. Results obtained when the heat treated clay was used to catalyze the oligomerization of $C_{14}$ alpha-olefin are shown in Table V, below. Table VI, showing the results when the clay is not heat treated, is offered for comparison.

TABLE V

| Sample No. | Liquid Flow (CC) | Temp (C) | Reactor Press. (psig MPa) | Time Since Prior Sample (H) | Total Time | C(%) | M(%) | D(%) | T+(%) | D/T Ratio |
|---|---|---|---|---|---|---|---|---|---|---|
| 1 | 16 | 153 | 300 | 63 | (63) | 76.0 | 24.0 | 50.1 | 25.9 | 1.93 |
| 2 | 16 | 155 | 300 | 9 | (72) | 44.0 | 56.0 | 32.1 | 11.9 | 2.70 |
| 3 | 16 | 155 | 300 | 15 | (87) | 80.4 | 19.6 | 50.0 | 29.8 | 1.69 |
| 4 | 19 | 155 | 300 | 9 | (96) | 80.1 | 19.9 | 50.7 | 29.3 | 1.73 |
| 5 | 19 | 156 | 300 | 15 | (11) | 80.3 | 19.7 | 51.3 | 29.0 | 1.77 |
| 6 | 19 | 156 | 300 | 9 | (120) | 82.1 | 17.9 | 42.4 | 34.8 | 1.36 |
| 7 | 19 | 156 | 300 | 15 | (135) | 81.6 | 18.4 | 48.1 | 33.4 | 1.44 |
| 8 | 19 | 156 | 300 | 8 | (143) | 81.6 | 18.4 | 49.2 | 32.4 | 1.52 |
| 9 | 19 | 156 | 300 | 16 | (159) | 81.1 | 18.9 | 49.8 | 31.3 | 1.59 |
| 10 | 19 | 156 | 300 | 9 | (168) | 80.2 | 19.8 | 49.2 | 31.0 | 1.59 |
| 11 | 19 | 156 | 300 | 63 | (231) | 81.2 | 18.8 | 49.9 | 31.2 | 1.60 |
| 12 | 19 | 156 | 300 | 9 | (240) | 79.9 | 20.3 | 52.5 | 27.2 | 1.93 |
| 13 | 19 | 156 | 300 | 15 | (255) | 78.4 | 21.6 | 55.9 | 22.4 | 2.49 |
| 14 | 19 | 156 | 300 | 9 | (264) | 78.0 | 22.0 | 55.8 | 22.2 | 2.51 |
| 15 | 16 | 156 | 300 | 15 | (279) | 79.0 | 21.0 | 58.2 | 25.8 | 2.06 |
| 16 | 16 | 156 | 300 | 9 | (288) | 72.5 | 22.5 | 56.1 | 21.5 | 2.61 |
| 17 | 20 | 156 | 300 | 15 | (303) | 79.0 | 21.0 | 51.6 | 22.4 | 1.88 |

TABLE V

| Sample No. | Liquid Flow (CC) | Temp (C) | Reactor Press. (psig MPa) | Time Since Prior Sample (H) | Total Time | C(%) | M(%) | D(%) | T+(%) | D/T Ratio |
|---|---|---|---|---|---|---|---|---|---|---|
| 18 | 20 | 156 | 300 | 9 | (312) | 72.9 | 22.1 | 52.3 | 25.8 | 2.03 |
| 19 | 20 | 156 | 300 | 15 | (327) | 76.9 | 23.1 | 52.4 | 24.5 | 2.14 |
| 20 | 20 | 156 | 300 | 9 | (336) | 75.5 | 24.5 | 52.2 | 23.3 | 2.24 |
| 21 | 20 | 156 | 300 | 63 | (399) | 73.5 | 26.4 | 51.9 | 21.7 | 2.39 |
| 22 | 20 | 156 | | 9 | (408) | 68.7 | 31.1 | 51.0 | 17.7 | 2.88 |

TABLE VI

| Sample No. | Liquid Flow (CC) | Temp (C) | Reactor Press. (psig MPa) | Time Since Prior Sample (H) | Total Time | C(%) | M(%) | D(%) | T+(%) | D/T Ratio |
|---|---|---|---|---|---|---|---|---|---|---|
| 1 | 20 | 155 | 300 | 8 | 8 | 73.5 | 26.5 | 47.1 | 26.3 | 1.75 |
| 2 | 20 | 155 | 300 | 14 | 14 | 77.1 | 22.9 | 46.8 | 30.5 | 1.53 |
| 3 | 20 | 155 | 300 | 14 | 30 | 77.3 | 22.7 | 48.0 | 28.3 | 1.71 |
| 4 | 20 | 155 | 300 | 16 | 46 | 78.8 | 21.1 | 48.1 | 29.6 | 1.63 |
| 5 | 20 | 155 | 300 | 8 | 54 | 79.8 | 20.2 | 48.3 | 31.5 | 1.53 |
| 6 | 20 | 155 | 300 | 15 | 69 | 79.9 | 20.1 | 47.1 | 32.8 | 1.44 |
| 7 | 20 | 155 | 300 | 9 | 78 | 79.6 | 20.4 | 47.6 | 32.0 | 1.49 |
| 8 | 20 | 155 | 300 | 63 | 141 | 78.2 | 21.3 | 49.9 | 28.5 | 1.75 |
| 9 | 20 | 155 | 300 | 9 | 150 | 77.2 | 22.8 | 51.2 | 26.1 | 1.96 |
| 10 | 20 | 155 | 300 | 15 | 165 | 75.9 | 24.2 | 51.6 | 24.2 | 2.13 |
| 11 | 20 | 156 | 300 | 9 | 174 | 73.7 | 26.3 | 52.7 | 21.0 | 2.15 |
| 12 | 20 | 156 | 300 | 15 | 189 | 78.9 | 27.1 | 50.0 | 22.9 | 2.18 |
| 13 | 20 | 156 | 300 | 9 | 198 | 72.8 | 27.7 | 49.9 | 22.4 | 2.23 |
| 14 | 20 | 156 | 300 | 15 | 213 | 71.7 | 28.3 | 49.9 | 22.2 | 2.25 |
| 15 | 20 | 156 | 300 | 9 | 222 | 70.5 | 29.5 | 50.3 | 20.2 | 2.49 |
| 16 | 20 | 154 | 300 | 15 | 232 | 70.1 | 29.9 | 49.9 | 20.2 | 2.47 |
| 17 | 24 | 155 | 300 | 7 | 244 | 62.2 | 32.8 | 47.3 | 19.9 | 2.38 |
| 18 | 24 | 155 | 300 | 65 | 309 | 62.2 | 37.7 | 45.5 | 16.8 | 2.71 |
| 19 | 24 | 155 | 300 | 90 | 318 | 59.1 | 40.9 | 43.9 | 15.2 | 2.89 |
| 20 | 24 | 155 | 300 | 15 | 333 | 56.8 | 43.2 | 44.0 | 22.7 | 1.94 |

TABLE VI

| Sample No. | Liquid Flow (CC) | Temp (C) | Reactor Press. (psig MPa) | Time Since Prior Sample (H) | Total Time | C(%) | M(%) | D(%) | T+(%) | D/T Ratio |
|---|---|---|---|---|---|---|---|---|---|---|
| 21 | 24 | 155 | 300 | 9 | 342 | 56.5 | 43.5 | 41.8 | 14.7 | 2.84 |
| 22 | 24 | 155 | 300 | 15 | 357 | 54.1 | 45.9 | 42.5 | 11.6 | 2.66 |
| 23 | 24 | 155 | 300 | 9 | 366 | 52.0 | 48.0 | 40.7 | 11.2 | 3.57 |
| 24 | 24 | 155 | 300 | 15 | 381 | 50.7 | 49.3 | 40.1 | 10.6 | 3.78 |
| 25 | 24 | 155 | 300 | 9 | 390 | 49.6 | 50.4 | 38.1 | 11.5 | 3.31 |
| 26 | 24 | 155 | 300 | 15 | 405 | 50.2 | 49.8 | 32.9 | 12.4 | 3.06 |
| 27 | 24 | 155 | 300 | 9 | 414 | 47.3 | 52.7 | 37.8 | 9.47 | 3.99 |
| 28 | 24 | 154 | 300 | 63 | 417 | 48.2 | 51.8 | 32.8 | 10.4 | 3.63 |
| 29 | 24 | 155 | 300 | 9 | 486 | 51.4 | 48.6 | 34.6 | 7.17 | 4.83 |
| 30 | 24 | 155 | 300 | 15 | 501 | 49.4 | 50.6 | 37.1 | 12.3 | 3.02 |
| 31 | 19 | 155 | 300 | 9 | 510 | 45.8 | 54.2 | 36.7 | 9.10 | 4.03 |
| 32 | 20 | 155 | 300 | 15 | 525 | 45.5 | 54.4 | 36.1 | 9.38 | 3.85 |
| 33 | 20 | 155 | 300 | 9 | 534 | 44.9 | 55.1 | 35.6 | 8.21 | 4.47 |
| 34 | 20 | 155 | 300 | 15 | 549 | 43.5 | 56.3 | 36.7 | 7.89 | 4.51 |
| 35 | 20 | 155 | 300 | 8 | 557 | 42.3 | 57.7 | 34.3 | 7.94 | 4.32 |
| 36 | 19 | 155 | 300 | 16 | 573 | 41.4 | 58.6 | 34.2 | 7.28 | 4.65 |
| 37 | 19 | 155 | 300 | 9 | 582 | 39.2 | 60.8 | 32.4 | 6.80 | 4.76 |
| 38 | 19 | 155 | 300 | 63 | 645 | 38.7 | 61.3 | 31.8 | 6.93 | 4.59 |
| 39 | 19 | 155 | 300 | 9 | 654 | 44.1 | 55.9 | 35.5 | 8.57 | 4.14 |
| 40 | 19 | | | 15 | 661 | 37.5 | 62.5 | 30.7 | 6.75 | 4.55 |

In Tables V and VI above, conversion of $C_{14}$ alpha-olefin against time is shown for heat treated Harshaw/Filtrol Clay 124 (1.0 wt.% $H_2O$) and non-heat treated clay 124 (7.1 wt.% $H_2O$), respectively. The reaction conditions for both continuous reactions were 155°C, and 2.17 MPa, with an LHSV (Liquid Hourly Space Velocity) of 0.2. Table V demonstrates the increased activity and prolonged catalyst life achieved by heat treating the clay before its use as a catalyst, showing 80 to 82% conversion at 90 to 230 hours, and 72 to 79% conversion from 250 to 400 hours, total continuous reaction time. Table VI, on the other hand, shows that the non-heat treated clay was less active at peak activity (achieving 70 to 79% conversion), and that its activity decreased more rapidly (conversion dropped to 62% and then to below 50% by 400 hours, total continuous reaction time.)

The embodiment of the invention using mixtures of linear olefins will be illustrated by the following Examples 4 to 11, in which the following procedures were used:

Batch-Flask

Olefin and clay catalyst were charged to a flask equipped with a stirrer, thermometer, heating mantle, and a water-cooled condenser ($N_2$ purge). The mixture was vigorously stirred and heated at a desired temperature for the desired time. The mixture was then cooled to ambient temperature and filtered with suction. The liquid was analyzed by liquid chromatography. The results are detailed in Tables VII and IX.

Batch-Autoclave

Olefin and clay catalyst were changed to an autoclave. The autoclave was sealed and heated to the desired temperature for the desired time. The mixture was then cooled to ambient temperature and filtered with suction. The liquid was then analyzed by liquid chromatography. The results are shown in Table VII and IX.

Hydrogenation of Oligomer

An autoclave was charged with oligomer and finely powdered nickel catalyst. The autoclave was flushed with hydrogen and then pressurized to 7.0 MPa with hydrogen. The mixture was heated to 200°C and stirred at this temperature for 4 hours. The mixture was then repressurized with hydrogen to 13.9 MPa as needed. The mixture was then cooled to ambient temperature, the catalyst was filtered and the monomer was removed. The results are shown in Tables VIII and X.

TABLE VII

ACID CLAY CATALYZED OLEFIN OLIGOMERIZATION WITH INTERNAL/ALPHA-OLEFIN MIXTURES

| EXAMPLE NO. | OLEFIN | (g) | HARSHAW/FILTROL CATALYST | (g) | REACTOR | TIME (H) | TEMP (°C) | CON. (%) | M (%) | D (%) | T+ (%) | D/T+ RATIO |
|---|---|---|---|---|---|---|---|---|---|---|---|---|
| 4 | C-14A,16A (63%, 36%) | 400 | H/F Clay 113 | 40 | Clave | 4 | 180° | 84.8 | 15.2 | 49.9 | 34.9 | 1.43 |
| 5 | C-14A,16A<br>C-16I | 200<br>200 | H/F Clay 24 | 40 | Flask | 6 | 150 | 71.1 | 28.9 | 48.1 | 23.0 | 2.09 |
| 6 | C-14A,16A<br>C-16I | 200<br>200 | H/F Clay 24 | 40 | Clave | 4 | 180 | 80.1 | 19.9 | 50.8 | 29.3 | 1.73 |
| 7 | C-14A,16A<br>C-16I | 300 | H/F Clay 24 | 40 | Clave | 4 | 180 | 78.2 | 21.8 | 52.5 | 25.8 | 2.03 |

Con. = Conversion; M = Monomer; D = Dimer; T+ = Trimer, plus Tetramer, Pentamer, etc.
A = Alpha; I = Interest

TABLE VIII
PROPERTIES OF REDUCED OILGOMER BOTTOMS

| EXAMPLE NO. | Percent Remaining by TGA at 121°C | Viscosity at (cSt) 99°C | VI | Pour Point °C |
|---|---|---|---|---|
| 4 | 89.5 | 6.17 | 131 | -29 |
| 5 | 87.7 | 5.21 | 135 | -32 |
| 6 | 92.5 | 5.79 | 129 | -37 |
| 7 | 87.5 | 5.68 | 130 | -37 |

TGA = Thermogravimetric Analysis
VI = Viscosity Index

## TABLE IX

### ACID CLAY CATALYZED OLEFIN OLIGOMERIZATION USING RECYCLE OLEFIN

| EXAMPLE NO. | OLEFIN | (g) | CATALYST | (g) | REACTOR | TIME (H) | TEMP (°C) | CON. (%) | M (%) | D (%) | T+ (%) | D/T+ RATIO |
|---|---|---|---|---|---|---|---|---|---|---|---|---|
| 8 | 14A | 400 | H/F Clay-124 | 40 | Clave | 5 | 160 | 78.3 | 21.7 | 48.8 | 29.5 | 1.65 |
| 9 | 14A<br>14-recycle | 300<br>100 | H/F Clay-124 | 40 | Clave | 5 | 160 | 74.6 | 25.4 | 47.2 | 27.5 | 1.72 |
| 10 | 14A<br>14-recycle | 300<br>100 | H/F Clay-124 | 40 | Flask | 5 | 160 | 73.7 | 25.3 | 48.5 | 26.2 | 1.85 |
| 11 | 14A<br>14-recycle | 300<br>100 | H/F Clay-124 | 40 | Flask | 5 | 160 | 74.9 | 25.1 | 45.2 | 29.7 | 1.52 |

## TABLE X

### PROPERTIES OF REDUCED OLIGOMER BOTTOMS

| EXAMPLE NO. | wt.% Recycle | Percent Remaining by TGA 121°C | Viscosity (cSt) | VI | Pour Point °C |
|---|---|---|---|---|---|
| 8 | 0 | 88.5 | 6.61 | 121 | -29 |
| 9 | 25 | 85.9 | 5.14 | 127 | -34 |
| 10 | 25 | 83.0 | 5.09 | 127 | -37 |
| 11 | 25 | 80.5 | 4.99 | 126 | -37 |

Examples 12 to 16 detailed in Table IX below demonstrate the embodiment of the present invention in which $C_{10}$-$_{24}$ linear olefins and propylene are co-oligomerized in batch reaction systems:

Co-oligomerization Procedure

1-Tetradecene (90 g) and catalyst (10 g Harshaw/Filtrol Clay grade 24) were charged to a 1 litre autoclave, and the autoclave was sealed. Propylene was pressurized in and the mixture was heated at 160°C for five hours. The pressure dropped slowly during the run as propylene was reacted. At the end of the run, the mixture was cooled to ambient temperature, a small amount of propylene was vented, and the mixture was filtered with suction. Analysis of the reaction mixture by high pressure liquid chromatography showed the presence of dimers, trimers, and higher oligomers. Percentage conversions obtained and dimer/trimer ratios are shown in Table XI.

Hydrogenation of Co-oligomers

An autoclave was charged with finely powered nickel catalyst and co-oligomer prepared according to the procedure outlined above. The autoclave was flushed with hydrogen and then pressurized to 7 MPa with hydrogen. The mixture was heated to 200°C and stirred at this temperature for 4 hours. The mixture was repressurized with hydrogen to 13.9 MPa as needed. The mixture was then cooled to ambient temperature, the catalyst was filtered and the monomer was removed. Properties of the reduced co-oligomers are recorded in Table XI below.

TABLE XI

CO-OLIGOMERIZATION OF PROPYLENE AND 1-TETRADECENE USING H/F CLAY-24

| EX. NO. | PROPYLENE (% BY WT. OF OLEFIN MIXTUREO | % CON OF $C_{14}$ ALPHA-OLEFIN | D/T+ RATIO | TGA-% REMAINING AT 250°C | VIS AT 99°C (Cst) | VI | POUR POINT (°C) |
|---|---|---|---|---|---|---|---|
| 12 | 5 (5 g) | - | - | 85.0 | 5.13 | 133 | -37 |
| 13 | 10 (10 g) | 61.6 | 2.87 | 79.0 | 4.67 | 123 | -40 |
| 14 | 15 (15 g) | 52.4 | 4.50 | 81.5 | 4.68 | 122 | -37 |
| 15 | 20 (20 g) | 43.8 | 6.02 | 84.3 | 4.44 | 115 | -40 |
| 16 | 0 | 78.3 | 1.65 | 88.5 | 6.61 | 121 | -29 |

Con. = Conversion; D = Dimer; T+ = Trimer, plus Tetramer, Pentamer, etc; TGA = Thermogravimetric Analysis; Vis. = Viscosity; and VI = Viscosity Index.

Properties were determined on reduced bottoms.

Examples 17 to 23 detailed in Table XII below demonstrate the embodiment of the present invention in which 1,3-diisopropenyl benzene or alpha-methylstyrene are used in batch reaction systems:

Co-oligomerization Procedure

Reactants and catalyst (10 wt.%, Harshaw/Filtrol Clay 124) were charged to a three-necked flask equipped with an overhead stirrer, a water cooled condenser, a heating mantle and a nitrogen purge. The mixture was heated to the desired temperature for the desired period of time (with vigorous stirring.) At the end of the reaction, the mixture was cooled to ambient temperature and filtered with suction. Analysis of the reaction mixture by high pressure liquid chromatography showed the presence of dimers, trimers, and higher oligomers. Percent conversions and dimer/trimer ratios are shown in the table below.

Hydrogenation of Co-oligomers

An autoclave was charged with finely powdered nickel catalyst (5 wt.%) and co-oligomer prepared according to the procedure outlined above. The autoclave was flushed with hydrogen and then pressurized to 7 MPa with hydrogen. The mixture was heated to 200°C and stirred at this temperature for 4 hours. The mixture was repressurized with hydrogen to 13.9 MPa as needed. The mixture was then cooled to ambient temperature, the catalyst was filtered and the monomer was removed under vacuum (approx. 130 Pa). Properties of the reduced co-oligomers are recorded in Table XII below.

TABLE XII

CO-OLIGOMERIZATION OF 1,3-DI-ISOPROPENYL BENZENE AND 1-TETRADENCENE USING H/F CLAY-124

| EX. NO. | OLEFIN FEED | % BY WT. OF OLEFIN MIXTURE | CATALYST | % CON. OF $C_{14}$ ALPHA-OLEFIN | D/T+ RATIO | TGA - % REMAINING AT 250°C | VIS. AT 99°C (cSt) | VI | POUR POINT °C |
|---|---|---|---|---|---|---|---|---|---|
| 17 | C-14A<br>1,3-DIPB | 90<br>10 | Clay-124 | 66.2 | 1.66 | 92.6 | 8.62 | 112 | -37 |
| 18 | C-14A<br>1,3-DIPB | 95<br>5 | Clay-124 | 73.5 | 1.41 | 86.5 | 6.35 | 119 | -34 |
| 19 | C-14A<br>1,3-DIPB | 90<br>10 | Heat-Treated Clay-124 | 36.3 | 3.37 | 90.0 | 11.9 | 101 | -32 |
| 20 | C-14A<br>1,3-DIPB | 95<br>5 | Heat-Treated Clay-124 | 50.2 | 2.92 | 86.5 | 6.63 | 119 | -37 |
| 21 | C-14A<br>AMS | 95<br>5 | Clay-124 | 75.1 | 1.90 | 87.1 | 5.66 | 132 | -37 |
| 22 | C-14A<br>AMS | 95<br>5 | Heat-Treated Clay-124 | 67.8 | 1.85 | 82.8 | 5.84 | 119 | -32 |
| 23 | C-14A | 100 | Clay-124 | 78.3 | 1.65 | 88.5 | 6.61 | 121 | -29 |

Con. = Conversion; D = Dimer; T+ = Trimer, plus Tetramer, Pentamer, etc; TGA = Thermogravimetric Analysis; Vis. = Viscosity Index; A = Alpha; 1,3-DIPB = 1,3-Di-isopropenyl benzene; and AMS = Alpha Methyl Styrene.

Examples 24 to 31 detailed in Table XIII below demonstrate the embodiment of the present invention in which linear and vinylidene olefins are co-oligomerized in batch reaction systems:

Procedure

Olefin and Harshaw/Filtrol Clay-13 catalyst (40 g) were charged to a flask equipped with a stirrer, thermometer, heating mantle, and a water-cooled condenser ($N_2$ purge). The mixture was vigorously stirred and heated at a desired temperature for the desired time. The mixture was then cooled to ambient temperature and filtered with suction. The liquid was analyzed by liquid chromatography. Olefin conversion and dimer/trimer ratio were determined and are detailed in the table below.

Hydrogenation of Oligomer

An autoclave was charged with oligomer and finely powdered nickel catalyst. The autoclave was flushed with hydrogen and then pressurized to 7 MPa with hydrogen. The mixture was heated to 200°C and stirred at this temperature for 4 hours. The mixture was repressurized with hydrogen to 13.9 MPa as needed. The mixture was then cooled to ambient temperature, the catalyst was filtered and unreacted monomer was removed. Properties of the resulting synthetic lubricant base stocks are shown in Table XIII below.

TABLE XIII

CO-OLIGOMERIZATION OF VINYLIDENE OLEFIN AND ALPHA- OR INTERNAL-OLEFIN[4]

| EX. NO. | OLEFIN | (g) OF OLEFIN | WT.% OLEFIN AS VINYLIDENE | TIME/TEMP (Hr)/(°C) | CON. (%) OF | D/T+ RATIO | Properties After Hydrogenation VIS. @99°C | VIS. INDEX | NOACK (%) | CCSIM (cp) | POUR POINT |
|---|---|---|---|---|---|---|---|---|---|---|---|
| 24 | 1416[1] | 400 | 20 | 5/160 | 81.4 | 1.58 | 5.43 | 139 | 13.7 | 800 | -29 |
| 25 | 1416 | 400 | 20 | 2/160<br>2/180 | 82.3 | 1.60 | 5.59 | 135 | 14.3 | 855 | -32 |
| 26 | 1416 | 400 | <5 | 5/160 | 85.0 | 1.28 | 6.08 | 140 | 12.0 | 880 | -23 |
| 27 | 1416 | 400 | <5 | 2/160<br>2/180 | 84.0 | 1.13 | 6.04 | 119 | 10.0 | 1015 | -21 |
| 28 | 16[2] | 400 | <5 | 2/160 | 84.0 | 1.32 | 7.17 | 137 | 8.3 | 1550 | -9 |
| 29 | 16 / 14-vin | 380/ 20 | 5 | 2/160<br>2/180 | 81.1 | 1.64 | 6.46 | 140 | 7.4 | 1077 | -15 |
| 30 | 16 / 14-vin | 360/ 40 | 10 | 2/160<br>2/180 | 91.6 | 0.79 | 5.92 | 142 | 8.4 | 888 | -18 |
| 31 | 1518I[3]/ 14-vin | 320/ 80 | 20 | 5/160 | 42.6 | 4.67 | 5.53 | 123 | * | * | -29 |

Vin. = vinylidene; Con. = conversion; I = Internal; Vis. = Viscosity; * = not determined; D = Dimer; T+ = Trimer + Tetramer + Pentamer, etc.; CCSIM = cold crank simulation; and cp = centipoise.

[1] From Ethyl Corporation: 1.3% $C_{12}$; 64.7% $C_{14}$; 33.0% $C_{16}$; and 1.0% $C_{18}$; containing approx. 19% of total oelfin in vinylidene form.
[2]From Shell Chemical Co.: approx. 55% $C_{14}$ and 45% $C_{16}$; containing approx. 94.9% in alpha form and less than 5% in vinylidene form.
[3]Shell Chemical Co. Neodene 1518 Internal Olefin: 1.8% $C_{14}$ and lower; 25.3% $C_{15}$; 26.3% $C_{16}$; 24.2% $C_{17}$; 18.5% $C_{18}$; and 3.9 % $C_{19}$.
[4]The catalyst for each of examples 24 to 31 was Harshaw/Filtrol Clay-13 (40g).

Example 32

The following Example 32 demonstrates the use of the present invention in continuous reaction systems. A 500 cc stainless steel reactor was charged with Harshaw/Filtrol Clay-24 and then heated for three days with a nitrogen purge (to drive off $H_2O$). The reactor was cooled to reaction temperature (155°C) and a 1416 $\alpha$ olefin mixture containing 20% of vinylidene olefin (determined by NMR) was pumped through the catalyst bed at a rate of 80 g/hour.

Initially, the reactor effluent showed 77.7% conversion and a dimer/trimer+ ratio of 1.44. After four weeks, the conversion had dropped to 37.5%. Monomer was distilled off to give 23 litres of material. The "bottoms" product was reduced with nickel catalyst to give a lubricant with the following properties:

| | |
|---|---|
| Viscosity (99°C) | 5.32 cSt |
| Viscosity Index | 120 |
| Pour Point (°C) | -46 |
| CCSIM (-20 °C) | 859 cp |
| Noack (250 °C) | 14.4% |

The embodiment of the invention in which a Lewis acid is deposited on the catalyst will be illustrated by the following Examples, 33 to 45, detailed below in Table XIV in which the following procedures were used:

Aluminium Nitrate Treatment

To 100 cc of Harshaw/Filtrol Grade 24 granules was added 1 litre of 0.5 N aqueous aluminium nitrate solution. The mixture was stirred mechanically for two days at room temperature. The solids were then filtered off and washed with distilled water until aluminium ions could no longer be detected in the washings. The remaining solids were then dried under a vacuum at 40°C. 108 g of white solid were recovered. Analysis showed the presence of 5.9% aluminium

Other Lewis Acid Treatments

100 cc samples of Harshaw/Filtrol Grade 24 granules were also treated, using the procedure detailed above, with aqueous solutions of the following Lewis Acids:

| Lewis Acid | Analysis of Treated Clay |
|---|---|
| $AlCl_3$ | Al, 6.5 % |
| $ZrCl_4$ | Zr, 3.8 % |
| $NiCl_2$ | Ni, 0.2 % |
| $ZnCl_2$ | Zn, 1.3 % |
| $TiCl_4$ | Ti, 5.1 % |

Batch Oligomerizations

Olefin and clay catalyst were charged to a flask equipped with a stirrer, thermometer, heating mantle, and a water-cooled condenser ($N_2$ purge). The mixture was vigorously stirred and heated to a desired temperature for the desired time. The mixture was then cooled to ambient temperature and filtered with suction.

The liquid was analyzed by liquid chromatography. The results are detailed in Table XIV.

TABLE XIV

OLEFIN OLIGOMERIZATION USING ACIDIC CLAYS TREATED WITH ALUMINIUM NITRATE AND OTHER LEWIS ACIDS

| EX. NO. | OLEFIN | (g) | CATALYST | (g) | TIME (H.) | TEMP. (°C) | CON. (%) | M (%) | D (%) | T+ (%) | D/T+ RATIO |
|---|---|---|---|---|---|---|---|---|---|---|---|
| 33 | C-14A | 100 | H/F Clay-24 | 10.0 | 5.0 | 160 | 81.0 | 19.0 | 48.7 | 32.3 | 1.51 |
| 34 | C-14A | 100 | $Al(NO_3)_3$ on H/F-24 | 10.0 | 5.0 | 160 | 85.0 | 15.0 | 45.2 | 39.8 | 1.14 |
| 35 | C-14A | 100 | $Al(NO_3)_3$ on H/F-24 | 5.0 | 5.0 | 160 | 75.0 | 25.0 | 46.9 | 27.1 | 1.73 |
| 36 | C-14A | 100 | H/F Clay-24 | 10.0 | 6.0 | 150 | 76.8 | 23.2 | 47.0 | 29.1 | 1.62 |
| 37 | C-14A | 100 | $Al(NO_3)_3$ on H/F-24 | 10.0 | 6.0 | 150 | 80.2 | 19.8 | 47.5 | 32.8 | 1.45 |
| 38 | C-14A | 100 | $Al(NO_3)_3$ on H/F-24 | 10.0 | 4.0 | 180 | 82.3 | 17.7 | 50.8 | 31.5 | 1.61 |
| 39 | C-14A | 100 | H/F Clay-24 | 10.0 | 5.0 | 160 | 78.3 | 21.7 | 46.8 | 31.5 | 1.49 |
| 40 | C-1518-10[1] | 100 | H/F Clay-24 | 10.0 | 5.0 | 180 | 67.4 | 32.6 | 47.3 | 19.1 | 2.48 |
| 41 | C-1510-IO | 100 | $Al(NO_3)$ on H/F-24 | 10.0 | 4.0 | 180 | 70.5 | 29.5 | 48.7 | 21.8 | 2.23 |
| 42 | C-14A | 100 | $NiCl_2$ on H/F-24 | 10.0 | 5.0 | 160 | 82.3 | 17.7 | 45.9 | 36.4 | 1.26 |
| 43 | C-14A | 100 | $ZnCl_2$ on H/F-24 | 10.0 | 5.0 | 160 | 83.2 | 16.8 | 43.1 | 37.6 | 1.15 |
| 44 | C-14A | 100 | $ZnCl_4$ on H/F-24 | 10.0 | 5.0 | 160 | 69.8 | 30.2 | 40.0 | 29.8 | 1.34 |
| 45 | C-14 | 100 | $AlCl_3$ on HF-24 | 10.0 | 5.0 | 160 | 80.3 | 19.7 | 48.4 | 31.9 | 1.52 |

Con.=Conversion; M = Monomer; D = Dimer; T+ = Trimer, plus Tetramer, Pentamer etc.
A = Alpha

[1] Shell Chemical Co. Neodene (R) 1518 Internal Olefin: 1.8% $C_{14}$ and lower; 25.3% $C_{15}$; 26.3% $C_{16}$; 24.2% $C_{17}$; 18.5 $C_{18}$; and 3.9 $C_{19}$.

## Claims

1. A process for the preparation of oligomers or co-oligomers of linear olefins containing from 10 to 24 carbon atoms in which an olefin composition comprising said olefins is contacted with a catalyst, characterized in that the catalyst is an acidic calcium montmorillonite clay having a moisture content up to 20 wt.%, a residual acidity of up to 30 mg KOH/g, and a surface area of 300 $M^2/g$ or greater.

2. A process according to Claim 1 characterized in that the linear olefins comprise alpha-olefins, internal olefins, or a mixture thereof.

3. A process according to Claim 1 or 2 characterized in that the olefin composition comprises said linear olefins and up to 20 weight % of propylene.

4. A process according to Claim 1 or 2 characterized in that the olefin composition comprises said linear olefins and up to 20 weight % of 1,3-di-isopropenyl benzene.

5. A process according to Claim 1 or 2 characterized in that the olefin composition comprises said linear olefins and a vinylidene olefin having 10 to 24 carbon atoms.

6. A process according to Claim 5 characterized in that the olefin composition contains 5 to 40 weight % of the vinylidene olefin.

7. A process according to any one of Claims 1 to 6 characterized in that the clay is one on which at least 0.01 wt % of a Lewis Acid selected from aluminium nitrate, zinc chloride, and nickel chloride has been deposited.

8. A process according to Claim 7 characterized in that 0.01 to 10 wt % of aluminium nitrate is deposited on the clay.

9. A process according to any one of Claims 1 to 8 characterized in that the olefin composition is contacted with the clay at a temperature of 150 to 180°C.

10. A process according to any one of Claims 1 to 9 characterized in that the oligomerized product is hydrogenated.

## Patentansprüche

1. Verfahren zur Herstellung von Oligomeren oder Co-Oligomeren linearer Olefine, die von 10 bis 24 Kohlenstoffatome enthalten, in dem eine Olefinzusammensetzung, die besagte Olefine umfaßt, mit einem Katalysator in Kontakt gebracht wird, dadurch gekennzeichnet, daß der Katalysator ein saurer Calcium-Montmorillonit-Ton mit einem Feuchtigkeitsgehalt bis zu 20 Gew.-%, einer Restacidität von bis zum 30 mg KOH/g und einer Oberfläche von 300 $m^2/g$ oder mehr ist.

2. Verfahren nach Anspruch 1, dadurch gekennzeichnet, daß die linearen Olefine Alpha-Olefine, innere Olefine oder eine Mischung derselben umfassen.

3. Verfahren nach Anspruch 1 oder 2, dadurch gekennzeichnet, daß die Olefinzusammensetzung besagte lineare Olefine und bis zu 20 Gew.-% Propylen umfaßt.

4. Verfahren nach Anspruch 1 oder 2, dadurch gekennzeichnet, daß die Olefinzusammensetzung besagte lineare Olefine und bis zu 20 Gew.-% 1,3-Diisopropenylbenzol umfaßt.

5. Verfahren nach Anspruch 1 oder 2, dadurch gekennzeichnet, daß die Olefinzusammensetzung besagte lineare Olefine und ein Vinyliden-Olefin mit 10 bis 24 Kohlenstoffatomen umfaßt.

6. Verfahren nach Anspruch 5, dadurch gekennzeichnet, daß die Olefinzusammensetzung 5 bis 40 Gew.-% des Vinyliden-Olefins enthält.

7. Verfahren nach einem der Ansprüche 1 bis 6, dadurch gekennzeichnet, daß der Ton einer ist, auf dem wenigstens 0,01 Gew.-% einer Lewis-Säure, die ausgewählt ist aus Aluminiumnitrat, Zinkchlorid und Nickelchlorid, abgeschieden worden sind.

8. Verfahren nach Anspruch 7, dadurch gekennzeichnet, daß 0,01 bis 10 Gew.-% Aluminiumnitrat auf dem Ton abgeschieden sind.

9. Verfahren nach einem der Ansprüche 1 bis 8, dadurch gekennzeichnet, daß die Olefinzusammensetzung mit dem Ton bei einer Temperatur von 150 bis 180°C in Kontakt gebracht wird.

10. Verfahren nach einem der Ansprüche 1 bis 9, dadurch gekennzeichnet, daß das oligomerisierte Produkt hydriert wird.

## Revendications

1. Procédé de préparation d'oligomères ou de co-oligomères d'oléfines linéaires contenant de 10 à 24 atomes de carbone, dans lequel on met en contact une composition d'oléfines comprenant lesdites oléfines avec un catalyseur, caractérisé en ce que le catalyseur est une argile de montmorillonite de calcium acide dont la teneur en humidité va jusqu'à 20 % en poids, l'acidité résiduelle jusqu'à 30 mg KOH/g, et dont l'aire de surface est de 300 $m^2/g$ ou plus.

2. Procédé selon la revendication 1 caractérisé en ce que les oléfines linéaires comprennent des alpha-oléfines, des oléfines internes, ou leur mélange.

3. Procédé selon la revendication 1 ou 2 caractérisé en ce que la composition d'oléfines comprend lesdites oléfines linéaires et jusqu'à 20 % en poids de propylène.

4. Procédé selon la revendication 1 ou 2 caractérisé en ce que la composition d'oléfines comprend lesdites oléfines linéaires et jusqu'à 20 % en poids de 1,3-di-isopropényl benzène.

5. Procédé selon la revendication 1 ou 2 caractérisé en ce que la composition d'oléfines comprend lesdites oléfines linéaires et une oléfine de vinylidène contenant 10 à 24 atomes de carbone.

6. Procédé selon la revendication 5 caractérisé en ce que la composition d'oléfines contient 5 à 40 % en poids d'oléfine de vinylidène.

7. Procédé selon l'une quelconque des revendications 1 à 6 caractérisé en ce que l'argile est une argile sur laquelle au moins 0,01 % en poids d'un acide de Lewis choisi parmi le nitrate d'aluminium, le chlorure de zinc, et le chlorure de nickel a été déposé.

8. Procédé selon la revendication 7 caractérisé en ce que 0,01 à 10 % en poids de nitrate d'aluminium est déposé sur l'argile.

9. Procédé selon l'une quelconque des revendications 1 à 8 caractérisé en ce qu'on met en contact la composition d'oléfines avec l'argile à une température de 150 à 180 °C.

10. Procédé selon l'une quelconque des revendications 1 à 9 caractérisé en ce que le produit oligomérisé est hydrogéné.